# EUROPÄISCHE PATENTSCHRIFT

(11) **EP 4 240 653 B1**
(45) Veröffentlichungstag und Bekanntmachung des Hinweises auf die Patenterteilung: **17.07.2024**
(21) Anmeldenummer: 21758639.5
(22) Anmeldetag: 05.08.2021
(51) Int. Cl.: B65B 3/00, A61L 2/02, B65B 7/28, B65B 55/19

(54) **VERSCHLIESSVORRICHTUNG ZUM VERSCHLIESSEN PHARMAZEUTISCHER BEHÄLTNISSE**
CLOSING APPARATUS FOR CLOSING PHARMACEUTICAL CONTAINERS
APPAREIL DE FERMETURE POUR FERMER DES RÉCIPIENTS PHARMACEUTIQUES

(30) Priorität: 05.11.2020 DE 102020129169
(43) Veröffentlichungstag der Anmeldung: 13.09.2023
(73) Patentinhaber: Syntegon Technology GmbH, 71332 Waiblingen (DE)
(72) Erfinder: BERGER, Tobias, 74564 Crailsheim (DE); KRAUSS, Ulrich, 74532 Ilshofen (DE); ILGENFRITZ, Markus, 91555 Feuchtwangen (DE)
(74) Vertreter: DREISS Patentanwälte PartG mbB
(86) Internationale Anmeldenummer: PCT/EP2021/071895
(87) Internationale Veröffentlichungsnummer: WO 2022/096169

(56) Entgegenhaltungen:
- CN-A- 107 670 081
- US-A- 5 641 004
- US-A1- 2011 094 619
- US-A1- 2020 338 765
- US-B2- 10 067 151

## Beschreibung

Die Erfindung betrifft eine Verschließvorrichtung zum Verschließen pharmazeutischer Behältnisse, mit einem Verschließwerkzeug zum Fügen eines Verschlusselements mit einem pharmazeutischen Behältnis, mit einer Fluideinheit zur Beaufschlagung des Behältnisses mit mindestens einem gasförmigen Fluid, wobei die Fluideinheit mindestens einen Filter aufweist.

Verschließvorrichtungen der vorstehend genannten Art sind aus der US 2011/094619 A1 und der US 5 641 004 A bekannt und werden insbesondere zum Verschließen von Spritzen, Karpulen und Vials verwendet. Wenn solche Behältnisse mit einem hochreaktiven Füllgut gefüllt sind, besteht die Gefahr, dass dieses Füllgut mit Sauerstoff reagiert. Um dies zu vermeiden, werden teilgefüllte Behältnisse mit einem Schutzgas beaufschlagt, verbunden mit dem Ziel, dass nach dem Fügen oder Aufsetzen eines Verschlusselements auf das Behältnis möglichst wenig Sauerstoff in dem Behältnis (also oberhalb des Füllspiegels des Füllguts) verbleibt. Um zu vermeiden, dass Fremdkörper mit dem Schutzgas in das Behältnis hinein transportiert werden, wird das Schutzgas durch einen entsprechenden Filter geleitet. Weitere Verschließvorrichtungen sind aus der US 10 067 151 B2 und aus der US 2020/338765 A1 bekannt.

Es ist auch bekannt, den Raum des Behältnisses oberhalb des Füllspiegels des Füllguts mit Unterdruck zu beaufschlagen, beispielsweise um die Einleitung des Schutzgases zu vereinfachen. Sofern es sich bei dem Füllgut um einen toxischen Wirkstoff handelt, ist es für den Fall des Einsatzes eines Unterdruckgases oder eines Teilvakuums bekannt, einen behälternahen Filter zu verwenden, der verhindert, dass toxische Gase aus dem Behälter heraus in einen Außenbereich, also beispielsweise in dem Bereich einer Unterdruckversorgung, gelangen.

Um unter den vorstehend genannten Bedingungen das Einbringen von Keimen in das Behältnis während des Schließvorgangs zu vermeiden, ist es erforderlich, Fluidleitungen der Fluideinheit und den mindestens einen Filter zunächst außerhalb einer Verschließstation zu sterilisieren. Die sterilisierten Teile werden dann in einen Reinraumbereich einer Verschließstation eingebracht und dort mittels Handschuheingriff montiert. Dieser Vorgang muss bei der Umstellung auf ein neues Füllgut jedes Mal wiederholt werden, insbesondere, wenn zuvor toxisches Füllgut verarbeitet wurde.

Der Ausbau, die Sterilisierung und der Einbau einer Vielzahl von Teilen (Fluidleitungen, Filter) über einen Handschuheingriff ist zeitaufwändig und fehleranfällig. Auch unter Gesichtspunkten des Arbeitsschutzes ist das vorstehend erläuterte und im Stand der Technik praktizierte Vorgehen nicht optimal.

Hiervon ausgehend liegt der vorliegenden Erfindung die Aufgabe zugrunde, eine Verschließvorrichtung anzugeben, welche eine vereinfachte Handhabung ermöglicht.

Diese Aufgabe durch eine Verschließvorrichtung mit den Merkmalen des Patentanspruchs 1 gelöst.

Bei der erfindungsgemäßen Verschließvorrichtung ist eine als Modul ausgebildete Werkzeugeinheit vorgesehen. Dieses Modul ermöglicht die gleichzeitige Handhabung von Verschließwerkzeug, Fluideinheit und Filter. Das Modul kann gemeinsam mit den vorstehend genannten Bauteilen an eine Werkzeugaufnahme angeschlossen oder von dieser gelöst werden, und zwar mittels einer Kopplungseinrichtung. Mit anderen Worten: Der Montage- und Demontagevorgang beschränkt sich darauf, die Werkzeugeinheit über die Kopplungseinrichtung mit der Werkzeugaufnahme zu verbinden bzw. die Werkzeugeinheit von der Werkzeugaufnahme zu lösen. Eine von der Werkzeugaufnahme entfernte Werkzeugeinheit kann außerhalb einer Verschließstation gehandhabt, insbesondere gereinigt werden, wobei es möglich ist, dass in diesem von der Werkzeugaufnahme entfernten Zustand des Moduls einzelne Bauteile von dem Modul entfernt werden, beispielsweise der mindestens eine Filter.

Die Werkzeugaufnahme kann ebenfalls dekontaminiert werden, insbesondere durch einfache Durchströmung mit einem Dekontaminationsmittel, beispielsweise mit Wasserstoffperoxid. Eine weitere Sterilisierung ist nicht erforderlich, da die Bauteile der Werkzeugeinheit sterilisiert werden, da die Werkzeugeinheit den mindestens einen Filter trägt und da dieser Filter im gekoppelten Zustand der Werkzeugeinheit mit der Werkzeugaufnahme das Einbringen von Keimen in einen Bereich fluidabwärts des mindestens einen Filters verhindert.

Bei einer bevorzugten Ausführungsform weist die Kopplungseinrichtung ein Kopplungselement und eine Kopplungselementaufnahme auf, welche in einem miteinander gekoppelten Zustand formschlüssig zusammenwirken. Eines der Bauteile, beispielsweise das Kopplungselement, ist der Werkzeugaufnahme zugeordnet, während das andere Bauteil, beispielsweise die Kopplungselementaufnahme, der Werkzeugeinheit zugeordnet ist. Auch eine umgekehrte Anordnung ist möglich. Der Formschluss der genannten Bauteile der Kopplungseinrichtung ermöglicht eine einfache und genaue Verbindung der Werkzeugeinheit mit der Werkzeugaufnahme, sodass eine ggf. automatisierte Bewegung der Werkzeugaufnahme in einfacher Weise auf eine entsprechende Bewegung der Werkzeugeinheit übertragbar ist.

Bevorzugt ist es ferner, dass die Werkzeugaufnahme eine Betätigungseinrichtung zum Schalten der Kopplungseinrichtung zwischen einem Sperrzustand, in welchem das Modul mit der Werkzeugaufnahme verbunden ist, und einem Freigabezustand, in welchem das Modul von der Werkzeugaufnahme lösbar ist, umfasst. Auf diese Weise kann die Ansteuerung der Kopplungseinrichtung in den Bereich der Werkzeugaufnahme hinein verlagert werden, wodurch der Aufbau der Werkzeugeinheit vereinfacht wird.

Bevorzugt ist es ferner, dass das Modul mindestens einen Fluidanschluss aufweist, dass die Werkzeugaufnahme mindestens eine Fluidversorgungseinrichtung aufweist und dass der mindestens eine Fluidanschluss und die Fluidversorgungseinrichtung in einem Zustand, in welchem das Modul und die Werkzeugaufnahme miteinander verbunden sind, miteinander fluidwirksam verbunden sind. Mit anderen Worten: Durch die Ankopplung der Werkzeugeinheit an die Werkzeugaufnahme wird die Fluideinheit der Werkzeugeinheit gleichzeitig mit der Fluidversorgungseinrichtung verbunden, ohne dass es weiterer Fügevorgänge bedarf.

Es ist außerdem bevorzugt, dass die Werkzeugaufnahme einen Aktor zur Betätigung des Verschließwerkzeugs umfasst. Dies hat den Vorteil, dass die Werkzeugeinheit keine eigene Betätigungseinrichtung zur Betätigung des Verschließwerkzeugs benötigt; somit muss also auch keine Steuerung oder Energieversorgung für die Werkzeugeinheit bereitgestellt werden. Dies führt zu einem weiter vereinfachten Aufbau der Werkzeugeinheit und zu einer weiter vereinfachten Reinigbarkeit.

Erfindungsgemäß ist die Fluideinheit zur Beaufschlagung des Behältnisses mit mindesten zwei unterschiedlichen gasförmigen Fluiden ausgebildet. Dies bedeutet, dass die Werkzeugeinheit mindestens zwei Fluidanschlüsse aufweist, welche über die Fluidversorgungseinrichtung der Werkzeugaufnahme mit mindestens zwei unterschiedlichen gasförmigen Fluiden versorgt werden.

Erfindungsgemäß ist ferner vorgesehen, dass das Modul mindestens zwei Filter umfasst, die mit jeweils einem der unterschiedlichen gasförmigen Fluide zusammenwirken. Auch diese mindestens zwei Filter können gemeinsam mit dem Modul gehandhabt werden, also gemeinsam bei Demontage der Werkzeugeinheit von der Werkzeugaufnahme von der Verschließvorrichtung entfernt und anderenorts, ggf. mit Demontage der Filter, sterilisiert werden.

Bevorzugt ist eine Kombination von gasförmigen Fluiden in Form eines Unterdruckgases und in Form eines Schutzgases. Mit einem Unterdruckgas ist ein Vakuum oder Teilvakuum gemeint. Das Schutzgas ist ein Inertgas, beispielsweise Stickstoff.

Bevorzugt ist es ferner, wenn die Werkzeugaufnahme an einem Roboterarm gehalten ist. Dieser ermöglicht die Handhabung (im Sinne einer Positionierung) der Werkzeugaufnahme und - bei Kopplung der Werkzeugeinheit mit der Werkzeugaufnahme - auch die Handhabung der Werkzeugeinheit und somit insbesondere auch des Verschließwerkzeugs. Es ist möglich, dass ein solcher Roboterarm einen Innenraum begrenzt, in welchem Teile der Fluidversorgungseinrichtung, also insbesondere Fluidleitungen, ggf. auch Ventile oder weitere Einrichtungen, angeordnet sind. Somit können die im Bereich einer Verschließstation benachbart zu einem zu verschließenden pharmazeutischen Behältnis freiliegenden Oberflächen der Verschließvorrichtung auf die Außenfläche des Roboterarms, der Werkzeugaufnahme und der Werkzeugeinheit beschränkt werden. Dies ermöglicht eine stark vereinfachte Reinigung einer Verschließstation bzw. der Bauteile der Verschließvorrichtung.

Ferner ist es bevorzugt, wenn die Verschließvorrichtung eine Handhabungseinrichtung zur Handhabung des Moduls aufweist. Bei dieser Handhabungseinrichtung handelt es sich um eine zu dem vorstehend genannten Roboterarm separate Einrichtung, welche die Positionierung der Werkzeugeinheit relativ zu der Werkzeugaufnahme ermöglicht. Bei dieser Handhabungseinrichtung kann es sich beispielsweise um einen zweiten Roboterarm handeln.

Weitere Merkmale und Vorteile der Erfindung sind Gegenstand der nachfolgenden Beschreibung und der zeichnerischen Darstellung eines bevorzugten Ausführungsbeispiels.

In der Zeichnung zeigt
- Fig. 1: eine schematische Ansicht einer Ausführungsform einer Verschließvorrichtung;
- Fig. 2: eine perspektivische Ansicht einer Werkzeugaufnahme und einer Werkzeugeinheit der Verschließvorrichtung gemäß Fig. 1, in einem miteinander gekoppelten Zustand;
- Fig. 3: eine der Fig. 2 entsprechende Ansicht, in einem voneinander entkoppelten Zustand der Werkzeugaufnahme und der Werkzeugeinheit;
- Fig. 4: eine perspektivische Ansicht der Werkzeugeinheit;
- Fig. 5: eine geschnittene Ansicht der Werkzeugeinheit längs einer ersten Schnittebene; und
- Fig. 6: eine geschnittene Ansicht der Werkzeugeinheit längs einer zu der ersten Schnittebene winklig versetzten zweiten Schnittebene.

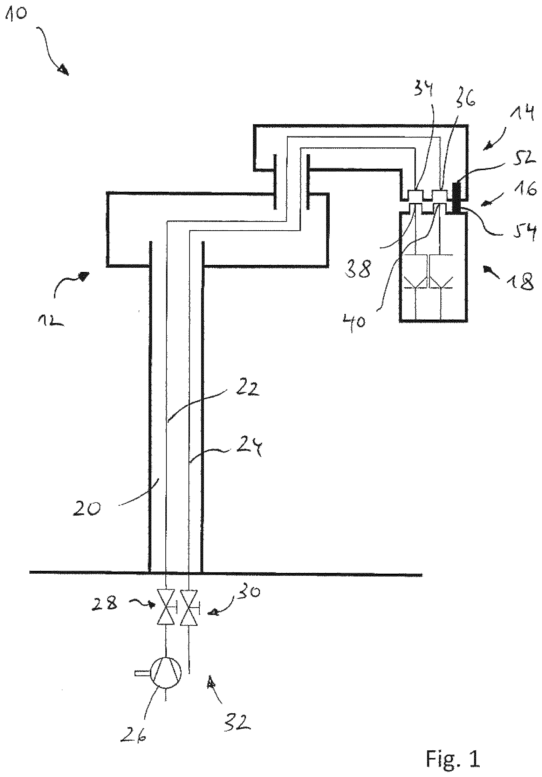

Eine Verschließvorrichtung zum Verschließen pharmazeutischer Behältnisse ist in der Zeichnung insgesamt mit dem Bezugszeichen 10 bezeichnet. Die Verschließvorrichtung 10 ist im Bereich einer Verschließstation einer Abfüllanlage angeordnet und insbesondere innerhalb eines Reinraums angeordnet. Die Verschließvorrichtung 10 weist einen in Figur 1 schematisch dargestellten Roboterarm 12 auf, der eine Werkzeugaufnahme 14 hält. Die Werkzeugaufnahme 14 ist über eine Kopplungseinrichtung 16 wiederholbar lösbar mit einer Werkzeugeinheit 18 verbindbar.

Der Roboterarm 12 weist einen Innenraum 20 auf, in welchem Fluidleitungen 22, 24 für unterschiedliche gasförmige Fluide angeordnet sind. Die Fluidleitung 22 dient zur Leitung von Vakuum. Zu diesem Zweck ist die Fluidleitung 22 mit einer Vakuumpumpe 26 verbindbar, und zwar unter Verwendung eines ersten Schaltventils 28. Ein zweites Schaltventil 30 dient dazu, ein Schutzgas, insbesondere Stickstoff, in die Fluidleitung 24 einzuleiten oder eine solche Einleitung zu sperren. Die Bauteile 20 bis 30 bilden gemeinsam eine Fluidversorgungseinrichtung 32, welche sich bis in einen der Kopplungseinrichtung 16 zugewandten Bereich der Werkzeugaufnahme 14 hinein erstreckt. Dort weist die Werkzeugaufnahme 14 jeweilige Versorgungsanschlüsse 34 bzw. 36 auf, die mit entsprechenden Fluidanschlüssen 38 bzw. 40 der Werkzeugeinheit 18 koppelbar sind.

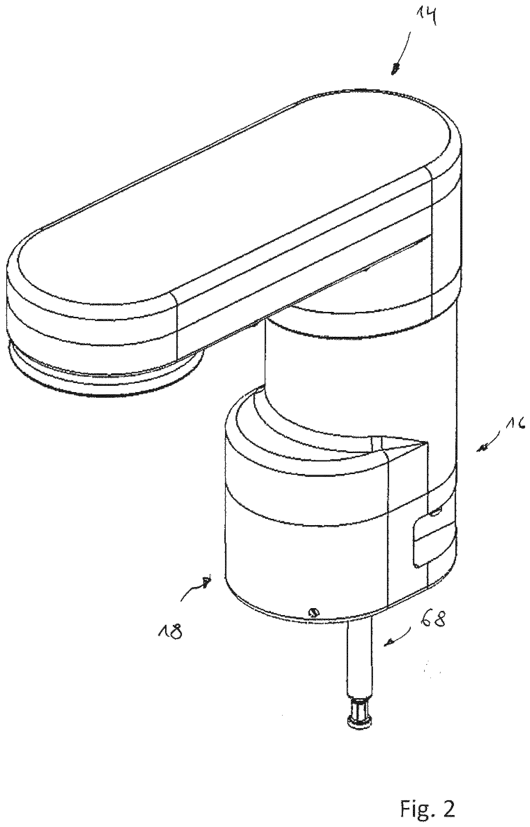

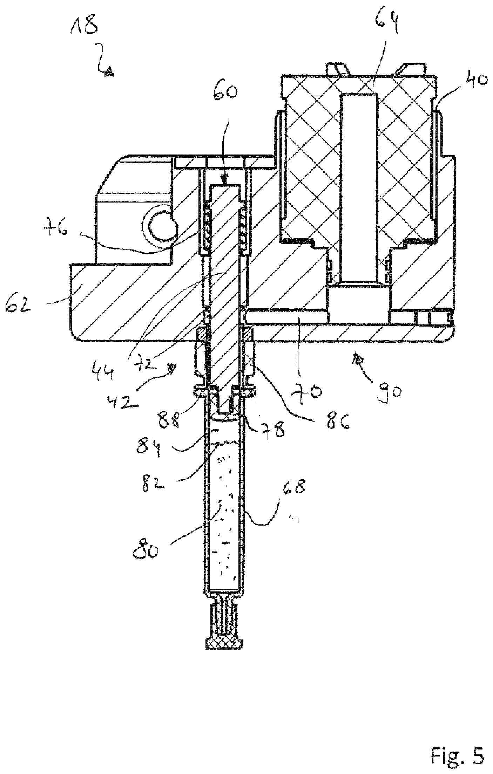

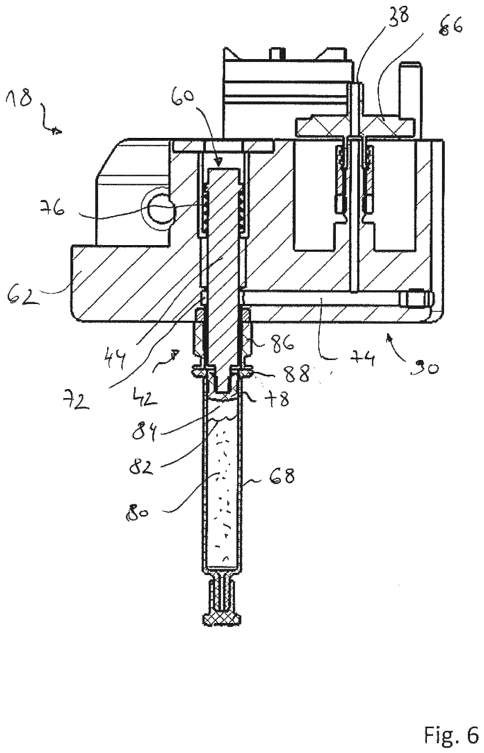

Der Aufbau der Werkzeugaufnahme 14, der Kopplungseinrichtung 16 und der Werkzeugeinheit 18 wird nachfolgend unter Bezugnahme auf die Figuren 2 bis 4 erläutert. Dabei zeigt die Figur 2 einen Zustand, in welchem die Werkzeugeinheit 18 mit der Werkzeugaufnahme 14 verbunden ist. Die Werkzeugeinheit 18 dient dazu, ein in den Figuren 2, 3, 5 und 6 dargestelltes pharmazeutisches Behältnis 68 mittels eines in den Figuren 5 und 6 dargestellten Verschließwerkzeugs 42 zu verschließen.

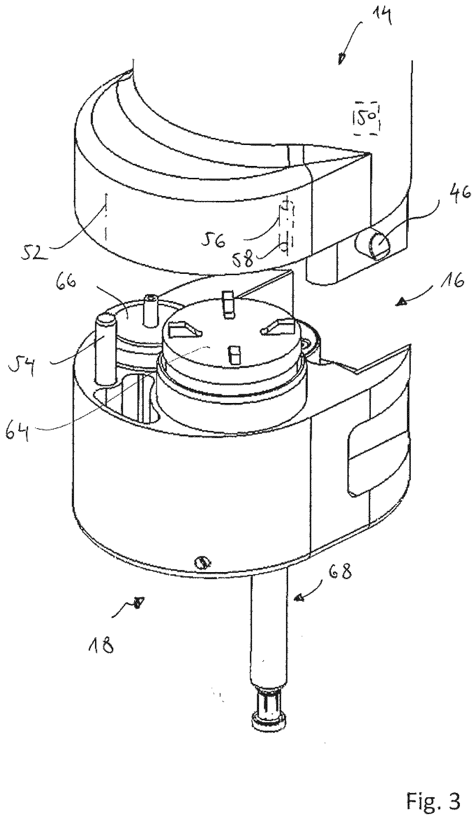

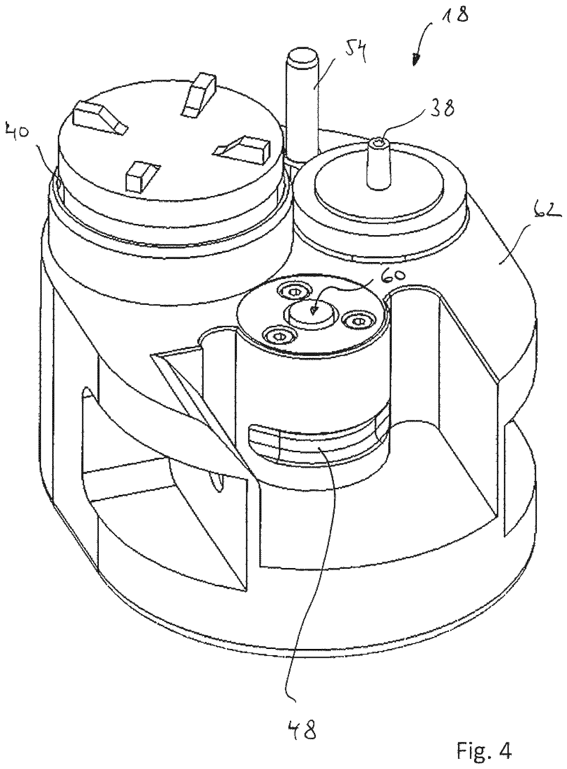

Die Kopplungseinrichtung 16 umfasst ein Kopplungselement 46, vergleiche Figur 3, welches in einem in Figur 2 dargestellten Sperrzustand der Kopplungseinrichtung 16 mit einer Kopplungselementaufnahme 48 der Werkzeugeinheit 18, vergleiche Figur 4, zusammenwirkt. Das Kopplungselement 46 ist beispielsweise als Bolzen ausgebildet, der mit einer Kopplungselementaufnahme 48 in Form einer Nut zusammenwirkt.

Das Kopplungselement 46 ist vorzugsweise an der Werkzeugaufnahme 14 angeordnet und mittels einer nur schematisch dargestellten Betätigungseinrichtung 50 betätigbar. Bei einer Betätigung des Kopplungselements 46 wird dieses relativ zu der Werkzeugaufnahme 14 und relativ zu der Kopplungselementaufnahme 48 bewegt, sodass die Kopplungseinrichtung einen Sperrzustand (vergleiche Figur 2) einnimmt oder einen Freigabezustand, vergleiche Figur 3.

Die Werkzeugaufnahme 14 umfasst ferner eine in Figur 3 mit einer strichpunktierten Linie angedeuteten Aufnahmebuchse 52, welche zur Aufnahme eines Stifts 54 der Werkzeugeinheit 18 dient. Die Verbindung zwischen den Bauteilen 52 und 54 unterstützt die vorstehend erläuterte formschlüssige Verbindung der Werkzeugeinheit 18 und der Werkzeugaufnahme 14 mittels der Kopplungseinrichtung 16.

Die Werkzeugaufnahme 14 umfasst ferner einen in Figur 3 mit gestrichelten Linien angedeuteten Aktor 56, der längs einer Aktorachse 58 bewegbar ist und somit eine Betätigungsfläche 60 des Schließwerkzeugs 42 betätigt, vergleiche Figuren 4, 5 und 6.

Der weitere Aufbau der Werkzeugeinheit 18 wird nachfolgend unter Bezugnahme auf die Figuren 4, 5 und 6 beschrieben.

Die Werkzeugeinheit 18 ist als Modul ausgebildet, welches ein Gehäuse 62 aufweist. Das Gehäuse 62 dient zur Anordnung und Positionierung nachfolgend beschriebener Bauteile. Das Gehäuse 62 umfasst insbesondere die bereits unter Bezugnahme auf Figur 1 genannten Fluidanschlüsse 38 und 40 sowie das Verschließwerkzeug 42. Ferner sind, bezogen auf die Fluidversorgungseinrichtung 32 stromabwärts der Fluidanschlüsse 38 und 40, jeweilige Filter 64 (vergleiche Figur 5) und 66 (vergleiche Figur 6) vorgesehen.

Ein erster Filter 64 ist als Sterilfilter ausgebildet. Mit dem Filter 64 wird vermieden, dass ggf. toxische Gase aus einem Innenraum eines zu verschließenden, pharmazeutischen Behälters 68 in den Bereich der Fluidversorgungseinrichtung 32 oder in einen Außenbereich der Verschließvorrichtung 10 gelangen. Der Filter 64 ist also dem Unterdruckgas bzw. Vakuum oder Teilvakuum der Fluidversorgungseinrichtung 32 zugeordnet.

Ein zweiter Filter 66 ist dem Schutzgas, also beispielsweise Stickstoff, zugeordnet. Der Filter 66 verhindert, dass Fremdkörper aus der Fluidversorgungseinrichtung 32 in das Behältnis 68 hinein eingebracht werden.

Der erste Filter 64 ist einem Fluidpfad zugeordnet, der über eine erste Stichleitung 70 zu einem ringförmigen Sammelraum 72 führt, vergleiche Figur 5. Für ein dem zweiten Filter 66 zugeleitetes Schutzgas ist eine entsprechende, zweite Stichleitung 74 vorgesehen, welche ebenfalls an dem Sammelraum 72 mündet, vergleiche Figur 6.

Der Sammelraum 72 erstreckt sich ringförmig um einen Stopfenstößel 44 des Verschließwerkzeugs 42 herum. Der Stopfenstößel 44 ist mittels einer Druckfeder 76 kraftbeaufschlagt, sodass der Stopfenstößel 44 in einer Grundstellung in Richtung der Werkzeugaufnahme 14 gehalten ist, sodass die Betätigungsfläche 60 - entsprechend Figur 4 - weiter von dem zu verschließenden Behältnis 68 entfernt angeordnet ist, als in einem in den Figuren 5 und 6 dargestellten Betätigungszustand. In diesem Betätigungszustand drückt der Aktor 56 auf die Betätigungsfläche 60, sodass der Betätigungsstößel 44 ein Verschlusselement 78, beispielsweise einen Stopfen, in einen offenen Endbereich des Behältnisses 68 hineindrückt.

Das Behältnis 68 ist beispielsweise in Form einer Spritze, einer Karpule oder eines Vials ausgebildet. Das Behältnis 68 ist vorzugweise an (in der Zeichnung nicht dargestellten) Halter so gehalten, dass ein zunächst offener und zu verschließender Endbereich des Behältnisses 68 bezogen auf die Richtung der Schwerkraft nach oben weist.

Das Behältnis 68 dient zur Aufnahme eines pharmazeutischen Füllguts 80, das einen oberen Füllspiegel 82 aufweist. Zwischen dem Füllspiegel 82 und einer oberen, zunächst offenen Begrenzung des Behältnisses 68 verbleibt ein Raum 84. Vor Einführung des Verschlusselements 78 in den zunächst offenen Endbereich des Behältnisses 68 ist das Verschlusselement 78 bereits an dem Stopfenstößel 44 aufgenommen. Der Stopfenstößel 44 befindet sich aber zunächst noch in seiner oberen Grundstellung (vgl. Fig. 4), in welcher die Betätigungsfläche 60 weiter oben angeordnet ist, sodass das Verschlusselement 68 zunächst noch außerhalb des Behältnisses 68 angeordnet ist.

Das Verschlusselement 78 ist dann zunächst noch auf Höhe eines Abdichtstutzens 86 angeordnet, der einenends mit der Werkzeugeinheit 18 verbunden und anderenends an einem endseitigen Flansch 88 des Behältnisses 68 anliegt. Der Abdichtstutzen 86 ist innen hohl und so bemessen, dass das Verschlusselement 78 einen kleineren Durchmesser aufweist, sodass gasförmiges Fluid aus dem Sammelraum 72 um das Verschlusselement 78 herum in den Raum 84 ein- und ausführbar ist. In diesem Zustand kann der Raum 84 unter Betätigung der Vakuumpumpe 76 und des ersten Schaltventils 78 mit Unterdruck beaufschlagt werden. Anschließend kann unter Betätigung des zweiten Schaltventils 30 Schutzgas in den Raum 84 eingeführt werden. Die vorstehend genannten Vorgänge können wiederholt werden, bis der Raum 84 möglichst sauerstoffarm ist und das Verschlusselement 78 durch Betätigung des Stopfenstößels 44 in das Behältnis 68 eingeführt werden kann, sodass das Behältnis 68 verschlossen wird. Das Verschlusselement 78 verbleibt an dem Behältnis 68.

Anschließend kann der Stopfenstößel mit einem weiteren Verschlusselement 78 gefügt und ein weiteres teilgefülltes Behältnis 68 bereitgestellt werden und die vorstehend beschriebenen Vorgänge wiederholt werden, bis die Abfüllung und das Verschließen einer Füllgutcharge abgeschlossen ist.

Die Bereiche der Werkzeugeinheit 18, welche bezogen auf die Fluidversorgungseinrichtung 32 stromabwärts der Filter 64 und 66, also dem Behältnis 68 zugewandt sind, werden insgesamt als Fluideinheit 90 bezeichnet. Diese Fluideinheit 90 umfasst insbesondere den Sammelraum 72 und Fluidleitungsbereiche, insbesondere in Form der Stichleitungen 70, 74.

Wie insbesondere aus Figur 3 ersichtlich, kann die Werkzeugeinheit 18 insgesamt als Modul gehandhabt werden. Dieses Modul umfasst die vorstehend genannte Fluideinheit 90, die Filter 64 und 66 sowie das Verschließwerkzeug 42 und einen Teil der Kopplungseinrichtung 16. Diese, in Figur 4 dargestellte Einheit, kann als Modul, also als Ganzes gehandhabt werden und mit der Werkzeugaufnahme 14 verbunden bzw. von dieser zwecks Sterilisierung zur Vorbereitung einer Folgecharge entfernt werden. Für diese Fügevorgänge kann eine (nicht dargestellte) weitere Handhabungseinrichtung verwendet werden, bspw. in Form eines zweiten Roboterarms.

## Patentansprüche

1. Verschließvorrichtung (10) zum Verschließen pharmazeutischer Behältnisse (68), mit einem Verschließwerkzeug (42) zum Fügen eines Verschlusselements (78) mit einem pharmazeutischen Behältnis (68), mit einer Fluideinheit (90) zur Beaufschlagung des Behältnisses (68) mit mindestens einem gasförmigen Fluid, wobei die Fluideinheit (90) mindestens einen Filter (64, 66) aufweist, **dadurch gekennzeichnet, dass** die Verschließvorrichtung (10) eine Werkzeugaufnahme (14) und eine Werkzeugeinheit (18) aufweist, wobei die Werkzeugeinheit (18) als Modul ausgebildet ist, welches mittels einer Kopplungseinrichtung (16) wiederholbar lösbar mit der Werkzeugaufnahme (14) verbindbar ist und welches das Verschließwerkzeug (42), die Fluideinheit (90) und den mindestens einen Filter (64, 66) umfasst, wobei die Fluideinheit (90) zur Beaufschlagung des Behältnisses (68) mit mindestens zwei unterschiedlichen gasförmigen Fluiden ausgebildet ist, wobei das Modul mindestens zwei Filter (64, 66) umfasst, die mit jeweils einem der unterschiedlichen gasförmigen Fluide zusammenwirken.

2. Verschließvorrichtung (10) nach Anspruch 1, **dadurch gekennzeichnet, dass** die Kopplungseinrichtung (16) ein Kopplungselement (46) und eine Kopplungselementaufnahme (48) aufweist, welche in einem miteinander gekoppelten Zustand formschlüssig zusammenwirken.

3. Verschließvorrichtung (10) nach einem der vorstehenden Ansprüche, **dadurch gekennzeichnet, dass** die Werkzeugaufnahme (14) eine Betätigungseinrichtung (50) zum Schalten der Kopplungseinrichtung (16) zwischen einem Sperrzustand, in welchem das Modul mit der Werkzeugaufnahme (14) verbunden ist, und einem Freigabezustand, in welchem das Modul von der Werkzeugaufnahme (14) lösbar ist, umfasst.

4. Verschließvorrichtung (10) nach einem der vorstehenden Ansprüche, **dadurch gekennzeichnet, dass** das Modul mindestens einen Fluidanschluss (38, 40) aufweist, dass die Werkzeugaufnahme (14) mindestens eine Fluidversorgungseinrichtung (32) aufweist und dass der mindestens eine Fluidanschluss (38, 40) und die Fluidversorgungseinrichtung (32) in einem Zustand, in welchem das Modul und die Werkzeugaufnahme (14) miteinander verbunden sind, miteinander fluidwirksam verbunden sind.

5. Verschließvorrichtung (10) nach einem der vorstehenden Ansprüche, **dadurch gekennzeichnet, dass** die Werkzeugaufnahme einen Aktor (56) zur Betätigung des Verschließwerkzeugs (42) umfasst.

6. Verschließvorrichtung (10) nach einem der vorstehenden Ansprüche, **dadurch gekennzeichnet, dass** ein gasförmiges Fluid ein Unterdruckgas ist und dass ein anderes gasförmiges Fluid ein Schutzgas ist.

7. Verschließvorrichtung (10) nach einem der vorstehenden Ansprüche, **dadurch gekennzeichnet, dass** die Werkzeugaufnahme (14) an einem Roboterarm (12) gehalten ist.

8. Verschließvorrichtung (10) nach einem der vorstehenden Ansprüche, **dadurch gekennzeichnet, dass** die Verschließvorrichtung (10) eine Handhabungseinrichtung zur Handhabung des Moduls aufweist.

## Claims

1. Closing apparatus (10) for closing pharmaceutical containers (68), having a closing tool (42) for joining a closure element (78) to a pharmaceutical container (68), having a fluid unit (90) for applying at least one gaseous fluid to the container (68), wherein the fluid unit (90) has at least one filter (64, 66), **characterized in that** the closing apparatus (10) has a tool receptacle (14) and a tool unit (18), wherein the tool unit (18) is designed as a module which is repeatedly detachable from and connectable to the tool receptacle (14) by means of a coupling device (16) and which comprises the closing tool (42), the fluid unit (90) and the at least one filter (64, 66), wherein the fluid unit (90) is designed to apply at least two different gaseous fluids to the container (68), wherein the module comprises at least two filters (64, 66), each of which interacts with one of the different gaseous fluids.

2. Closing apparatus (10) as claimed in claim 1, **characterized in that** the coupling device (16) has a coupling element (46) and a coupling element receptacle (48), which interact form-fittingly in a state coupled to one another.

3. Closing apparatus (10) as claimed in one of the preceding claims, **characterized in that** the tool receptacle (14) comprises an actuating device (50) for switching the coupling device (16) between a locking state, in which the module is connected to the tool receptacle (14), and a release state, in which the module is releasable from the tool receptacle (14).

4. Closing apparatus (10) as claimed in one of the preceding claims, **characterized in that** the module comprises at least one fluid connection (38, 40), **in that** the tool receptacle (14) comprises at least one fluid supply device (32), and **in that** the at least one fluid connection (38, 40) and the fluid supply device (32) are fluidically connected to each other in a state in which the module and the tool receptacle (14) are connected to each other.

5. Closing apparatus (10) as claimed in one of the preceding claims, **characterized in that** the tool receptacle comprises an actuator (56) for actuating the closing tool (42).

6. Closing apparatus (10) as claimed in one of the preceding claims, **characterized in that** one gaseous fluid is a negative-pressure gas, and **in that** another gaseous fluid is a protective gas.

7. Closing apparatus (10) as claimed in one of the preceding claims, **characterized in that** the tool receptacle (14) is held on a robot arm (12).

8. Closing apparatus (10) as claimed in one of the preceding claims, **characterized in that** the closing apparatus (10) has a handling device for handling the module.

## Revendications

1. Dispositif de fermeture (10) pour fermer des récipients pharmaceutiques (68), avec un outil de fermeture (42) pour assembler un élément de fermeture (78) avec un récipient pharmaceutique (68), avec une unité de fluide (90) pour alimenter le récipient (68) avec au moins un fluide gazeux, dans lequel l'unité de fluide (90) présente au moins un filtre (64, 66), **caractérisé en ce que** le dispositif de fermeture (10) présente un logement d'outil (14) et une unité d'outil (18), dans lequel l'unité d'outil (18) est réalisée sous la forme d'un module, lequel peut être relié de manière détachable et répétable au logement d'outil (14) au moyen d'un dispositif de couplage (16) et lequel comprend l'outil de fermeture (42), l'unité de fluide (90) et le au moins un filtre (64, 66), dans lequel l'unité de fluide (90) est réalisée pour alimenter le récipient (68) avec au moins deux fluides gazeux différents, dans lequel le module comprend au moins deux filtres (64, 66), qui coopèrent chacun avec l'un des fluides gazeux différents.

2. Dispositif de fermeture (10) selon la revendication 1, **caractérisé en ce que** le dispositif de couplage (16) présente un élément de couplage (46) et un logement d'élément de couplage (48), lesquels coopèrent par complémentarité de forme dans un état couplé l'un à l'autre.

3. Dispositif de fermeture (10) selon l'une quelconque des revendications précédentes, **caractérisé en ce que** le logement d'outil (14) comprend un dispositif d'actionnement (50) pour commuter le dispositif de couplage (16) entre un état de blocage, dans lequel le module est relié au logement d'outil (14), et un état de libération, dans lequel le module peut être détaché du logement d'outil (14).

4. Dispositif de fermeture (10) selon l'une quelconque des revendications précédentes, **caractérisé en ce que** le module présente au moins un raccord de fluide (38, 40), **en ce que** le logement d'outil (14) présente au moins un système d'alimentation en fluide (32) et **en ce que** le au moins un raccord de fluide (38, 40) et le système d'alimentation en fluide (32) sont reliés l'un à l'autre de manière à agir sur le fluide dans un état dans lequel le module et le logement d'outil (14) sont reliés entre eux.

5. Dispositif de fermeture (10) selon l'une quelconque des revendications précédentes, **caractérisé en ce que** le logement d'outil comprend un actionneur (56) pour actionner l'outil de fermeture (42).

6. Dispositif de fermeture (10) selon l'une quelconque des revendications précédentes, **caractérisé en ce qu'**un fluide gazeux est un gaz à pression négative et **en ce qu'**un autre fluide gazeux est un gaz de protection.

7. Dispositif de fermeture (10) selon l'une quelconque des revendications précédentes, **caractérisé en ce que** le logement d'outil (14) est retenu sur un bras robotisé (12).

8. Dispositif de fermeture (10) selon l'une quelconque des revendications précédentes, **caractérisé en ce que** le dispositif de fermeture (10) présente un système de manipulation pour la manipulation du module.
